# EUROPEAN PATENT SPECIFICATION

(11) **EP 2 617 360 B1**
(45) Date of publication and mention of the grant of the patent: **30.03.2016**
(21) Application number: 13151625.4
(22) Date of filing: 17.01.2013
(51) Int. Cl.: A61B 8/00, G06F 19/00

(54) **Probe device, server, system for diagnosing ultrasound image, and method of processing ultrasound image**
Sondenvorrichtung, Server, System zum Diagnostizieren eines Ultraschallbildes und Verfahren zum Verarbeiten eines Ultraschallbildes
Dispositif de sonde, serveur, système de diagnostic d'image ultrasonore et procédé de traitement d'image ultrasonore

(30) Priority: 17.01.2012 KR 20120005275
(43) Date of publication of application: 24.07.2013
(73) Proprietor: Samsung Electronics Co., Ltd, Gyeonggi-do 443-742 (KR)
(72) Inventor: Eum, Jae-young, Gyeonggi-do (KR); Cho, Jeong, Gangdong-gu (KR)
(74) Representative: D'Halleweyn, Nele Veerle Trees Gertrudis

(56) References cited:
- US-A1- 2004 030 585
- US-A1- 2005 049 495
- US-A1- 2007 239 019
- US-A1- 2010 298 711
- US-A1- 2011 034 209

## Description

The present invention relates to a probe device, a server, a system for diagnosing an ultrasound image, and a method of processing an ultrasound image. More particularly, the present invention relates to a probe device, server, a system for diagnosing an ultrasound image, and a method of processing an ultrasound image, all of which are based on cloud computing connected to a network.

Ultrasonic waves are sound waves in a frequency domain higher than an audible frequency domain (audible frequencies typically ranging from 20 Hz to 20 kHz). Accordingly, ultrasonic waves cannot be heard by people. A typical system for diagnosing an ultrasound image transmits an ultrasonic signal from a point of transmission exterior to the body, which penetrates into tissues or organs in a human body by using the human body as a medium, and obtains an image of the tissues or organs by using information about the ultrasonic signal reflected from the tissues or organs in the human body.

Such ultrasonic image diagnosing systems are typically small, inexpensive, and display the image in real-time. Also, since the system has high stability as it is not exposed to X-rays or the like, the system may be widely used with other image diagnostic apparatuses, such as an X-ray diagnostic apparatus, a computerized tomography (CT) scanner, a magnetic resonance image (MRI) apparatus, and a nuclear medicine diagnostic apparatus.

The ultrasonic system may comprise a cart type system or a hand carried ultrasonic (HCU) type system. Due to its larger size, it is more difficult to use a cart type system than an HCU type in emergency situations or common homes. Meanwhile, an HCU type system may be easier to use in emergency situations, etc., than the cart type because the HCU type is manufactured by using a transducer or an apparatus for processing ultrasound image information in a portable terminal shape. However, the quality of an ultrasound image of the HCU type system suffers, particularly when compared with the cart type systems, primarily due to limitation in size of a portable terminal and constraints on power usage, as the portable terminal uses rechargeable batteries.

US 2011/0034209 A1 discloses an imaging system, having: an imaging data acquisition device; a remote image reconstruction and data processing facility; and a wireless data transfer to transmit raw data from the data acquisition device to the remote facility. At the facility, the raw data is processed to prepare a diagnostic image that can be transmitted to an expert or non-expert, or transmitted back to the display of the wireless data transfer device.

US 2007/0239019 A1 discloses a portable ultrasonic imaging probe adapted to connect to a host computer via a passive interface cable, e.g., a standard USB 2.0 peripheral interface cable or a standard IEEE 1394 "Firewire" peripheral interface cable. In accordance with an embodiment, the portable ultrasound imaging probe includes a probe head, a logarithmic compressor, an envelope detector, and analog-to-digital converter and interface circuitry, all of which receive power from the host computer via the passive interface cable. To simplify the portable ultrasonic imaging probe, none of electronic beamforming, time gain compensation, gray-scale mapping and scan conversion are performed within the probe.

The present invention provides a probe device, a server, a system for diagnosing an ultrasound image, and a method of processing an ultrasound image. Embodiments of the present invention overcome limitations in terms of compactness and slimness. Also manufacturing costs may be remarkably reduced. Embodiments of devices for diagnosing an ultrasound image in accordance with the method, system and apparatus according to the present invention address the need to be compact and slim in order to increase the portability of the device and to overcome the problems that have heretofore prohibited development of such a high performance processing apparatus that processes a high resolution image signal or performs various functions of the device.

According to an exemplary aspect of the present invention, there is provided a probe device preferably including: a pulse generator including a pulser that generates a pulse signal; a transducer that converts the pulse signal generated by the pulse generator to ultrasonic waves, and converts received ultrasonic waves to an electrical signal; an analog signal processor that generates an echo signal by using the electrical signal converted by the transducer; and a probe communicator that communicates through a network with a server that executes an ultrasound image diagnostic application requested by an electronic device, and transmits the echo signal generated by the analog signal processor to the server.

The probe device may preferably further include a coupling unit that performs a process of interlocking (coupling) the probe device with the ultrasound image diagnostic application executed by the server.

The pulse generator may preferably further include a transmitter beam former that focuses ultrasonic waves output from the transducer.

The analog signal processor may preferably include: an amplifier that amplifies the electrical signal converted by the transducer; and an analog/digital (A/D) converter that converts the electrical signal amplified by the amplifier to a digital echo signal.

The analog signal processor may preferably further include a receiver beam former that focuses the digital echo signal converted by the A/D converter.

The analog signal processor may preferably further include a compressor that compresses a data size of the digital echo signal converted by the A/D converter.

The probe communicator may preferably include at least one of a mobile communication module that directly connects to the network, a wireless Internet module, a wired Internet module, and a local area communication module, or may include a communication interface connecting to the network through an external communication module.

The probe device may preferably further include a user input unit that receives input from a user.

According to another exemplary aspect of the present invention, there is provided a server preferably including: a server communicator that communicates with a probe device and an electronic device through a network; a data storage unit that stores an ultrasound image diagnostic application; and an operation unit that executes the ultrasound image diagnostic application when the electronic device requests to execute the ultrasound image diagnostic application, wherein the operation unit generates ultrasound image data by using an echo signal transmitted from the electronic device, and transmits the generated ultrasound image data to the electronic device.

According to another exemplary aspect of the present invention, there is provided a system for diagnosing an ultrasound image, the system preferably including: a probe device that transmits an echo signal to a server through a network; a server that executes an ultrasound image diagnostic application, wherein ultrasound image data is generated by using the echo signal received from the probe device; and an electronic device including a display unit that receives through a network and displays the ultrasound image data generated by the ultrasound image diagnostic application of the server.

The ultrasound image diagnostic application of the server may preferably execute a transmitter beam forming process for focusing the echo signal.

The ultrasound image diagnostic application of the server may execute a receiver beam forming process that focuses the digital echo signal converted by the A/D converter. The degree of focus can be according to a predetermined algorithm that determines focus of the digital echo signal.

The probe device may include a communication module directly connecting to the network, or may directly connect to the network. Here, the communication module may be built in or removable from the probe device.

Alternatively, the probe device may connect to the network through the electronic device by being connected to the electronic device wirelessly or via wires. For example, the probe device may include a communication interface connected to a mobile device to connect to the mobile device wirelessly or via wires, and the mobile device may connect to the network so that the probe device indirectly connect to the network.

The display unit of the electronic device may display a user interface of the ultrasound image diagnostic application executed by the server.

The probe device may be controllable by the electronic device.

An ultrasound image mode of the ultrasound image diagnostic application may be selected through the electronic device.

In addition, the electronic device may preferably include a first electronic device and a second electronic device, which respectively receive and display ultrasound image data generated by the ultrasound image diagnostic application of the server. The ultrasound image diagnostic application executed by the server may be controlled by either of the first and second electronic devices. Alternatively, the ultrasound image diagnostic application executed by the server may be controlled by both the first and second electronic devices. The ultrasound image data or the user interface of the ultrasound image diagnostic application displayed on the first electronic device may be manipulatable by the second electronic device connected through the server. For example, the first electronic device may be operated locally by a user such as an emergency rescuer or a self-diagnosing user, adjacently located to the probe device, and the second electronic device may be operated by an image diagnostic expert remotely located away from the probe device, such as at a hospital. Here, the ultrasound image diagnostic application of the server may be executed through the first electronic device, and the second electronic device may receive and display ultrasound image data generated by the ultrasound image diagnostic application of the server. It is also possible that invention could operate on a peer-to-peer basis with a second electronic device being associated with trained medical experts, a hospital or medical center, and the second electronic device may have more processing capabilities as compared with the first device. Of course, an ultrasound image mode of the ultrasound image diagnostic application executed in the server may be selected through the second electronic device.

The electronic device may comprise a mobile terminal connected to a wireless network or a desktop connected to a wired or wireless network. The mobile terminal may be a mobile phone, a smart phone, a touch pad, a laptop, a digital broadcasting terminal, a personal digital assistant (PDA), a portable multimedia player (PMP), a navigation device, a tablet personal computer (PC), or a remote controller.

The server may perform a user authentication process on the electronic device.

According to another exemplary aspect of the present invention, there is provided a method of processing an ultrasound image, the method preferably including: transmitting a request for executing an ultrasound image diagnostic application from an electronic device to a server through a network; executing the ultrasound image diagnostic application by using the server according to the request of the electronic device; generating an echo signal by using the probe device; transmitting the echo signal generated by the probe device to the server; generating ultrasound image data using the server by using the received echo signal; and displaying an ultrasound image on the electronic device by receiving the ultrasound image data from the server through the network.

The method may preferably further include interlocking the probe device with the ultrasound image diagnostic application executed by the server.

The generating of the echo signal may include: converting ultrasonic waves received from a material to be examined into an electrical signal; and converting the electrical signal into a digital echo signal.

The generating of the echo signal may further include compressing a data size of the digital echo signal.

A transmitter beam forming process of focusing ultrasonic waves output from a transducer of the probe device may be performed in the probe device. A receiver beam forming process of focusing ultrasonic waves output from a transducer of the probe device may be performed by the ultrasound image diagnostic application of the server.

The method may preferably further include displaying a user interface of the ultrasound image diagnostic application executed by the server and output to the electronic device, on the electronic device.

The probe device may be controllable by the electronic device.

An ultrasound image mode of the ultrasound image diagnostic application may be selected through the electronic device.

The method may preferably further include performing user authentication on the electronic device when the electronic device connects to the server or when the ultrasound image diagnostic application is executed by an operation unit of the server.

The method may preferably further include, when the electronic device that transmitted the request to execute the ultrasound image diagnostic application to the server is a first electronic device, preparing a second electronic device for displaying the ultrasound image by receiving the ultrasound image data from the server. The server may transmit the ultrasound image data to the second electronic device according to a request of the first or second electronic device. The method may preferably further include performing user authentication on the second electronic device, when the second electronic device connects to the server or to the ultrasound image diagnostic application.

The ultrasound image diagnostic application executed by the server may be controllable by either of the first and second electronic devices. The ultrasound image diagnostic application executed by the server may be controllable by both of the first and second electronic devices.

The ultrasound image or the user interface of the ultrasound image diagnostic application displayed on the first electronic device may be manipulatable from the second electronic device connected through the server. The ultrasound image or the user interface of the ultrasound image diagnostic application displayed on the second electronic device may be manipulatable from the first electronic device connected through the server.

### BRIEF DESCRIPTION OF THE DRAWINGS

The above and other exemplary features and advantages of the presently claimed invention will become more apparent to a person of ordinary skill in the art by describing in detail exemplary embodiments thereof with reference to the attached drawings in which:
FIG. 1 is a diagram of a system for diagnosing an ultrasound image, according to an exemplary embodiment of the present invention;
FIG. 2 is a block diagram of a probe device according to an exemplary embodiment of the present invention;
FIG. 3 is a block diagram of a server according to an exemplary embodiment of the present invention;
FIG. 4 is a block diagram of an electronic device according to an exemplary embodiment of the present invention;
FIG. 5 is an example of a user interface displayed on the electronic device of FIG. 4;
FIG. 6 is a flowchart for describing operations of a system for diagnosing an ultrasound image, according to an exemplary embodiment of the present invention;
FIG. 7 is a block diagram of a probe device according to another exemplary embodiment of the present invention;
FIG. 8 is a block diagram of a probe device according to another exemplary embodiment of the present invention;
FIG. 9 is a block diagram of a probe device according to another exemplary embodiment of the present invention;
FIG. 10 is a diagram of a system for diagnosing an ultrasound image, according to another exemplary embodiment of the present invention;
FIG. 11 is a flowchart illustrating a method of processing an ultrasound image, according to an exemplary embodiment of the present invention;
FIG. 12 is a diagram of a system for diagnosing an ultrasound image, according to another exemplary embodiment of the present invention; and
FIG. 13 is a diagram of a system for diagnosing an ultrasound image, according to another exemplary embodiment of the present invention.

### DETAILED DESCRIPTION OF THE INVENTION

Hereinafter, the present invention will be described more fully with reference to the accompanying drawings, in which exemplary embodiments of the invention are shown. In the drawings, like reference numerals denote like elements, and the sizes and thicknesses of elements may be exaggerated for clarity. As used herein, the term "and/or" includes any and all combinations of one or more of the associated listed items. Expressions such as "at least one of," when preceding a list of elements, modify the entire list of elements and do not modify the individual elements of the list. The examples provided herein are merely illustrative to assist a person of ordinary skill in the art with an understanding and appreciation of the claimed invention, and the appended claims are in no way limited to the examples provided herein.

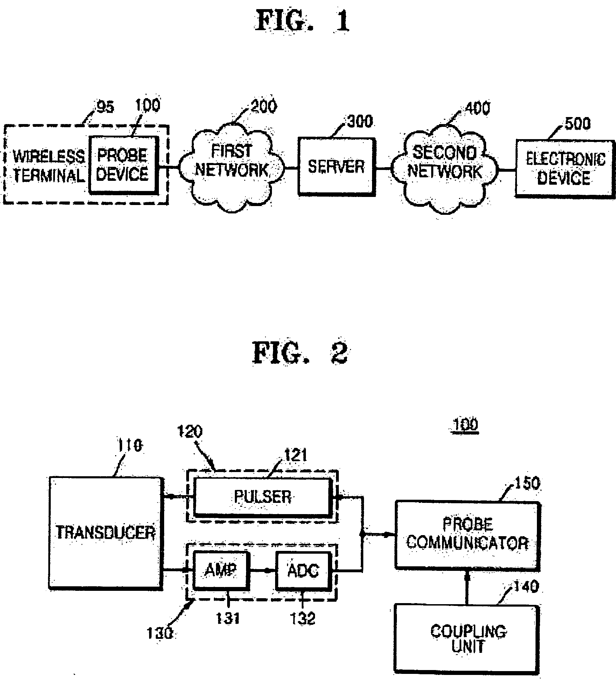

FIG. 1 is a diagram of a system for diagnosing an ultrasound image, according to an exemplary embodiment of the present invention.

Referring now to FIG. 1, the system may preferably include a probe device 100 for transmitting and receiving ultrasonic waves, a server 300 for executing an ultrasound image diagnostic application for generating an ultrasound image by using echo data received by the probe device 100, and an electronic device 500 for providing a user interface of the ultrasound image diagnostic application executed in the server 300. There may be one or more electronic devices 500.

The probe device 100 which may optionally be part of a wireless terminal 95, such as a mobile communication terminal and the server 300 transmit and receive data through a first network 200. Also, the server 300 and the electronic device 500 transmit and receive data through a second network 400. The first and second networks may include an Internet network, a mobile communication network, or the like.

The first and second networks 200 and 400 may be the same or different kinds of networks according to usage specifications. For example, the second network could be 802.11, WIFI, or WLAN with the server based for example, at a medical facility. While it is preferred and understood that cloud computing is used, it is also within the scope of the claimed invention that the electronic device 500 could be connected to the second server via wire, such as Ethernet. A person of ordinary skill in the art should understand and appreciate that the claimed invention may include the use of a proxy server in communication with server 300.

FIG. 2 is a block diagram of the probe device 100 according to the current exemplary embodiment of the present invention.

Referring now to FIG. 2, the probe device 100 preferably includes a transducer 110, a pulse generator 120, an analog signal processor 130, a coupling unit 140, and a probe communicator 150.

The transducer 110 preferably includes a plurality of conversion elements that convert and transmit a pulse signal generated by the pulse generator 120 to ultrasonic waves, and convert reflected ultrasonic waves to an analog echo signal, i.e., an electronic signal. The conversion elements may be formed of a piezoelectric material, and may have a one dimensional (1D) or two dimensional (2D) arrangement structure. The transducer 110 generates ultrasonic waves upon receiving a high voltage electric pulse from the pulse generator 120, and converts ultrasonic waves reflected from inside a body of a person to be examined back to an analog electrical signal (analog echo signal).

The pulse generator 120 includes a pulser 121 for generating a high voltage electric pulse signal.

The analog signal processor 130 generates a digital echo signal (echo data) by using the analog echo signal converted by the transducer 110. The analog signal processor 130 may preferably include an amplifier 131 amplifying the analog echo signal converted by the transducer 110, and an analog/digital (A/D) converter 132 converting the analog echo signal to a digital format.

The amplifier 131 may preferably include, for example, a low noise amplifier (LNA) for satisfactory sensitivity, or a time gain compensator (TGC) for adjusting a gain according to time to compensate for signal attenuation that increased while passing through a human body.

Besides the elements shown in FIG. 2, the probe device 100 may further include various elements, such as a battery, a high voltage multiplexer (HVMUX), an LNA, and a transmit/receive (T/R) switch, which are well-known to one of ordinary skill in the art.

The coupling unit 140 may perform a process of interlocking the probe device 100 to the ultrasound image diagnostic application executed in the server 300 through the first network 200. For example, the interlocking of the probe device to the ultrasound image diagnostic comprises the coupling unit 140 storing identification information of the probe device 100, and thus when the probe communicator 150 connects to the server 300, the identification information of the probe device 100 is transmitted to the server 300 so that the ultrasound image diagnostic application executed in the server 300 identifies the probe device 100.

The probe communicator 150 may include at least one of a mobile communication module, a wireless Internet module, and a local area communication module. The mobile communication module transmits and receives a wireless signal with at least one of a base station, an external terminal, and a server in a mobile communication network, such as 2G, 3G, or 4G. The probe communicator 150 is expected to be compatible with any future network wireless protocols beyond those previously listed. The wireless internal module is a module for connecting to a wireless Internet. The local area communication module is a module for local area communication, and enables at least one wireless communication method from among the standard IEEE 802.11 of a wireless network about a wireless local area network (LAN) suggested by the Institute of Electrical and Electronics Engineers (IEEE) and a wireless LAN including partial infrared ray communication, the standard IEEE 802.15 about a wireless personal area network (PAN) including Bluetooth, ultra wideband (UWB), and Zigbee, the standard 802.16 about a metropolitan area network (MAN) or broadband wireless access (BWA) including fixed wireless access (FWA), and the standard IEEE 802.20 about mobile Internet of mobile BWA (MBWA) including Wibro and WiMAX. The probe communicator 150 may further include a wired Internet module for wired Internet access to communicate with the server 300 via wire according to user environments. The probe communicator 150 communicates with the server 300 by using at least one of the mobile communication module, the wired Internet module, the wireless Internet module, and the local area communication module, and hereinafter, the probe device 100 communicates with the server 300 without repeating a detailed communication method such as the many communication protocols mentioned hereinabove.

A person of ordinary skill in the art should understand and appreciate that the probe device 100 and the server 300 may communicate with each other via the first network 200. For example, the first network 200 may perform a relay function for communication linkage with a plurality of external devices, or may include a relay device (not shown) that performs a relay function for communication linkage between a peripheral device and an external communication network (or a network). The relay device may perform a function of changing a protocol, if a communication protocol of the probe device 100 constituting a relay target and a communication protocol of the server 300 are different from each other. The relay device may be an access point, a gateway, a hotspot, a router, or a combination thereof, or a proxy. Alternatively, the probe device 100 may be directly connected to the server 300 via an ad hoc method or a Wi-Fi direct (WFD) method, without using a relay device.

The probe communicator 150 transmits the echo data processed by the analog signal processor 130 to the server 300 via the first network 200, and receives probe control data for controlling the probe device 100 back from the server 300.

The probe device 100 described above may comprise a portable wireless device for communicating with the server 300 by connecting to the first network 200 in a wireless manner.

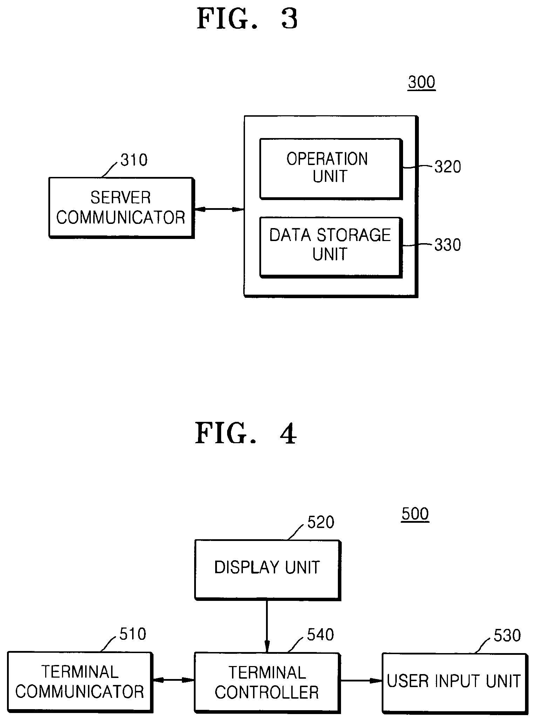

The probe communicator 150 according to the current embodiment includes a communication module, such as a mobile communication module, a wired Internet module, a wireless Internet module, or a local area communication module, but the probe communicator 150 is not limited thereto. In other words, the probe communicator 150 may only include a general-purpose communication interface (not shown), such as a universal serial bus (USB) or a Bluetooth, and the communication module, such as the mobile communication module, the wired Internet module, the wireless Internet module, or the local area communication module, may be an external communication module or a part of a mobile device, such as a smart phone. In this case, the probe communicator 150 may connect to the external communication module or the mobile device wirelessly or via wires and the external communication module or the mobile device may connect to the first network, such that the probe device 100 indirectly contact the first network 200. FIG. 3 is a block diagram of the server 300 according to the current exemplary embodiment of the present invention. Referring now to FIG. 3, the server 300 includes a server communicator 310, an operation unit 320, and a data storage unit 330. The server communication and operation unit comprise hardware and may include separate processors or microprocessors, or a single microprocessor.

The server communicator 310 may preferably include at least one of a mobile communication module, a wired Internet module, a wireless Internet module, and a local area communication module. Since the mobile communication module, the wired internet module, the wireless Internet module, and the local area communication module are substantially the same as those described above with respect to the probe communicator 150, details thereof will not be repeated.

Upon receiving a command to execute the ultrasound image diagnostic application through the server communicator 310, the operation unit 320 executes the ultrasound image diagnostic application by loading the ultrasound image diagnostic application from the data storage unit 330 into hardware such as a microprocessor controller. The ultrasound image diagnostic application includes an image processing module for generating an ultrasound image based on ultrasonic echo data obtained from the probe device 100. Also, the ultrasound image diagnostic application may provide a user interface to the electronic device 500. The user interface may not only include a menu related to processing an ultrasound image, but may also preferably include a control menu, etc. for controlling the probe device 100. Also, the ultrasound image diagnostic application may include a module for managing various types of diagnostic data related to an ultrasound image of a person to be examined. In addition, the ultrasound image diagnostic application may preferably further include an authentication module for the probe device 100 and/or the electronic device 500. The operation unit 320 may perform a process of identifying the probe device 100 to be connected, by comparing identification information transmitted from the probe device 100 and identification information of the probe device 100, which is pre-stored in the data storage unit 330. Also, the operation unit 320 may perform a user authentication process on the electronic device 500. The user authentication process may be performed on the electronic device 500 when the electronic device 500 is connected to the server 300 or when the ultrasound image diagnostic application is executed.

The data storage unit 330, which comprises a non-transitory machine readable medium, may store an execution program of the ultrasound image diagnostic application or may temporarily store data processed in the ultrasound image diagnostic application, such as digital echo data transmitted from the probe device 100 or ultrasound image data processed from the digital echo data. Also, the data storage unit 330 may store the identification information of the probe device 100 and identification information of the electronic device 500. Furthermore, the data storage unit 330 may store information about the person to be examined including identification information (ID, a resident registration number, a name, an address, a phone number, etc.), a diagnostic result of the person to be examined, etc. The data storage unit 330 may include a storage medium of at least one type, from among a flash memory type, a hard disk type, a multimedia card micro type, a card type memory (for example, a secure digital (SD) card or an extreme digital (XD) card), a random access memory (RAM) type, a static RAM (SRAM) type, a read-only memory (ROM) type, an electrically erasable programmable read-only memory (EEPROM) type, a programmable read-only memory (PROM) type, a magnetic memory type, a magnetic disk type, and an optical disk type, just to name some non-limiting examples. Such a data storage unit 330 may exist as a separate storage server connected to a network.

The server 300 may be understood as a cloud computing service server processor for executing an application requested by a client, such as the probe device 100 or the electronic device 500, through the first and second networks 200 and 400.

FIG. 4 is a block diagram of the electronic device 500 according to the current embodiment of the present invention. Referring now to FIGS. 1 and 4, the electronic device 500 may include a terminal communicator 510 for communicating with the server 300, a display unit 520 for displaying a screen of an image diagnostic application executed in the server 300, a user input unit 530 for inputting a manipulation menu of the image diagnostic application executed in the server 300, and a terminal controller 540 for generating a control message, or the like.

The terminal communicator 510 may preferably include at least one of a mobile communication module, a wired Internet module, a wireless Internet module, and a local area communication module. The mobile communication module, the wired Internet module, the wireless Internet module, and the local area communication module are substantially the same as those described above with respect to the probe communicator 150, and thus, details thereof are not repeated.

The display unit 520 displays the screen of the image diagnostic application executed in the server 300. For example, the screen of the image diagnostic application may be a user interface displayed with an ultrasound image processed by the server 300 and a manipulation menu for selecting a mode (for example, a brightness (B) mode, a Doppler (D) mode, a color (C) mode, a motion (M) mode, and an elastic mode) for processing an ultrasound image. The display unit 520 may include at least one of a liquid crystal display (LCD), a thin film transistor-liquid crystal display (TFT-LCD), an organic light-emitting diode (OLED), a flexible display, a transparent display, and a three dimensional (3D) display. The claimed invention is not limited to the types of aforementioned displays, as these are merely exemplary or preferable.

The user input unit 530 generates key input data that is input to manipulate the image diagnostic application executed in the server 300 by a user. The user input unit 530 may include a key pad or a touch pad (static pressure or electrostatic for example). Specifically, a touch screen is formed when the user input unit 530 that is a touch pad type forms a mutual layer structure with the display unit 520. The claimed invention is not limited by the types of touch screens disclosed herein, which are provided only for illustrative purposes.

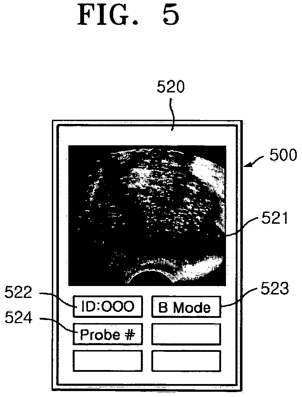

FIG. 5 is an example of a user interface displayed on the display unit 520 of the electronic device 500 of FIG. 4. Referring to FIG. 5, the display unit 520 may display an ultrasound image 521 obtained by outputting ultrasound image data generated by the server 300, information 522 about a user, information 523 about an ultrasound image processing mode, and identification information 524 about the probe device 100. When the user input unit 530 is activated with an input method such as a touch screen method, menus for the information 522, 523, and 524 may be directly manipulated to manipulate the image diagnostic application including the ultrasound image processing mode or to control the probe device 100.

Referring back to FIG. 4, the terminal controller 540 may generate a control message for controlling the image diagnostic application executed in the server 300, according to key input data input from the user input unit 530. The control messages can be communicated through a browser. More particularly, the wireless device electronic device 500 may have an "app", or a graphic user interface that has a link to activate the image diagnostic application. The electronic device 500 may further include a memory (not shown) for temporarily storing image data of the image diagnostic application input from the server 300.

As described above, the electronic device 500 according to the current exemplary embodiment operates as an input and output unit of the server 300 to display a screen for executing the image diagnostic application of the server 300 and input a manipulation menu. The electronic device 500 may be a mobile terminal connected to a wireless network or a desktop connected to a wired or wireless network. Examples of the mobile terminal include a mobile phone, a smart phone, a touch pad, a laptop, a digital broadcasting terminal, a personal digital assistant (PDA), a portable multimedia player (PMP), a navigation device, a tablet personal computer (PC), and a remote controller, just to name some non-limiting possibilities. Since processing of an ultrasound image with a high load is performed by the image diagnostic application of the server 300, the electronic device 500 does not need high performance and may be sufficient enough to display a process result of the server 300.

Next, operations of a system for diagnosing an ultrasound image, according to an embodiment of the present invention will be described.

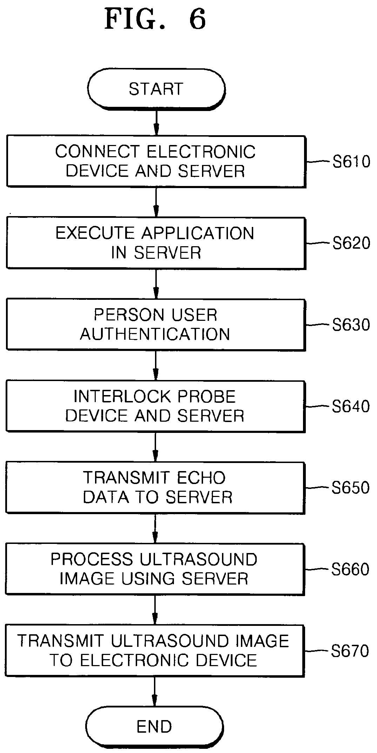

FIG. 6 is a flowchart for describing operations of a system for diagnosing an ultrasound image, according to an exemplary embodiment of the present invention. Referring to FIGS. 1 through 6, the system performs the following operations.

First, the electronic device 500 connects to the server 300, in operation S610. The electronic device 500 may comprise a terminal possessed by a medical image expert, such as a doctor, a nurse, a medical laboratory technologist, or an emergency rescuer, or a terminal possessed by a person to be examined. In the event where a person to be examined is using the probe, it could be plugged into or be in communication with the electronic device 500 wirelessly through a local communication protocol. The probe can also be integrated in the electronic device in such an instance. When a message requesting to execute the ultrasound image diagnostic application is transmitted from the electronic device 500 to the server 300, the server 300 executes the ultrasound image diagnostic application, in operation S620.

User authentication may be additionally performed in operation S630 while performing the ultrasound image diagnostic application. Operation S630 may be performed, while executing the ultrasound image diagnostic application, as the server 300 requests the electronic device 500 for identification information of a user, the electronic device 500 transmits input manipulation of the user or user identification information pre-input in the electronic device 500 to the server 300, and the server 300 compares the received identification information and the pre-stored user identification information. In some cases, the user authentication may be performed by using identification information of the electronic device 500. FIG. 6 shows that operation S630 is performed while executing the ultrasound image diagnostic application, but alternatively, operation S630 may be performed when the electronic device 500 initially connects to the server 300.

Next, the probe device 100 is turned on, and interlocked to the ultrasound image diagnostic application of the server 300, in operation S640. When the probe device 100 is turned on, the coupling unit 140 inside the probe device 100 transmits its identification information to the server 300, and the ultrasound image diagnostic application executed in the server may identify the probe device 100 based on the received identification information. A plurality of the probe device 100 may be connected to the server 300, and in this case, the ultrasound image diagnostic application of the server 300 may display the identification information of communicable probe devices 100 on the display unit 520 of the electronic device 500 for the user to select the probe device 100.

Then, the probe device 100 transmits digital echo data generated from reflected ultrasonic waves to the server 300, in operation S650. The ultrasound image diagnostic application of the server 300 generates ultrasound image data by using the echo data received from the probe device 100, in operation S660.

A process of processing the ultrasound image performed by the ultrasound image diagnostic application may be a well-known process. For example, the process may include a transmitter beam forming process, a receiver beam forming process, a filtering process, and a scan conversion process.

The transmitter beam forming process is a signal processing process of focusing ultrasonic waves output from the transducer 110 so as to view reflection characteristics of a tissue at a desired location, and determines pulse signals to be applied to each of conversion elements of the transducer 110 by considering locations of the conversion elements and a focus point of the output ultrasonic waves.

The receiver beam forming process is a process of focusing a received ultrasound echo signal to view reflection characteristics of a tissue at a desired location. As an example of the receiver beam forming process, an adaptive weight beamforming technique of assigning weights to echo signals converted by the conversion elements of the transducer 110 of FIG. 2 and performing a delay and sum (DAS) operation is well-known.

The filtering process may be bandpass filtering, and not only reduces noise, but is also used to process an ultrasound image using a reference frequency (provides excellent penetration) or secondary harmonic waves (provides excellent resolution due to excellent tissue classifying characteristics).

The scan conversion process is a process of converting a coordinate system of raw data into a coordinate system used in the display unit 520 of the electronic device 500.

The ultrasound image diagnostic application may employ various algorithms for a digital processing path to extract a clear image. For example, the ultrasound image diagnostic application may employ algorithms such as match filtering, time frequency compensation, echo line averaging, speckle reduction, frame smoothing, and edge detection.

Meanwhile, the ultrasound image diagnostic application may process the ultrasound image in at least one of a B mode, a D mode, a C mode, an M mode, and an elastic mode. Furthermore, the ultrasound image diagnostic application may process a 2D image or a 3D image.

More particularly, the B mode provides a black and white image used to examine tissue structure and organs. The D mode provides a speed of a moving object in a Doppler spectrum image by using a Doppler effect. The C mode provides a speed of a moving object in a color image by using a Doppler effect. The M mode provides bio-information (such as luminance information) of certain part of the object changing according to time in an image in the B mode. The elastic mode provides an image of a reaction difference when compression is applied and not applied to the object.

The server 300 transmits the generated ultrasound image data to the electronic device 500 in operation S670 so as to display the ultrasound image on the display unit 520 of the electronic device 500. Furthermore, the server 300 transmits the various modes for processing the ultrasound image or manipulation menu information about controlling the probe device 100 to the electronic device 500 so that the user selects a mode for processing the ultrasound image and controls the probe device 100 by manipulating the electronic device 500.

The system described above may be employed in plurality of different scenarios. For example, an emergency rescuer may execute the image diagnostic application of the server 300 through the electronic device 500 in an emergency, and scan a predetermined part of a person to be examined by interlocking the probe device 100 with the image diagnostic application while looking at an ultrasound image displayed on the electronic device 500, thereby checking the person on the spot in real-time.

Alternatively, a doctor or a patient, i.e., a person to be examined, may execute the image diagnostic application of the server 300 through the electronic device 500 at the house of the patient, and scan a predetermined part of the person by interlocking the probe device 100 with the image diagnostic application while looking at an ultrasound image displayed on the electronic device 500, thereby checking the person on the spot in real-time.

Moreover, an image diagnostic expert in a hospital may possess the probe device 100 and the electronic device 500, and diagnose a patient on the spot in real-time instead of a separate ultrasonic diagnostic room. Here, the ultrasound image obtained through the probe device 100 may be stored in the electronic device 500 and used for future diagnosis.

The probe device 100 and the plurality of electronic devices 500 connected to the server 300 may independently operate. In other words, one probe device 100 and one electronic device 500 may pair up to connect to the server 300. In this case, the server 300 may be placed at a hospital or in a data sensor, and an expensive apparatus for processing an ultrasound image may be replaced by the server 300, thereby reducing expenses for the system.

A cloud computing service denotes a service of providing computing resources requested by a user at any time anywhere through the Internet. In the cloud computing service, when a client requests to execute an application, a server executes the application and provides only a result to the client. In other words, the client may be considered as a type of input and output device in the cloud computing service. In the system of the current exemplary embodiment, since processing of an ultrasound image is separated from the probe device 100 and the electronic device 500, and performed by the ultrasound image diagnostic application of the server 300, the probe device 100 and the electronic device 500 may be understood as a type of client, and the server 300 may be understood as a cloud service server. High performance computing resources are required to obtain an ultrasound image and diagnostic results of high quality, but such a request for high performance computing resources is an obstacle to compact and reduce a thickness of the system. However, in the current exemplary embodiment, since the image diagnostic application for obtaining an ultrasound image from echo data is performed by the server 300, the probe device 100 may be compacted and slimmed down for portability, and thus an ultrasound image diagnostic service may be easily used at home and outdoors.

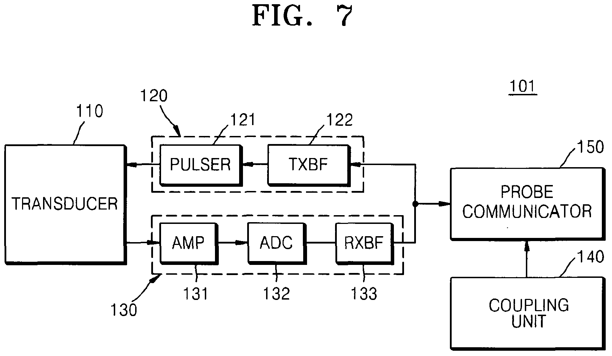

FIG. 7 is a block diagram of a probe device 101 according to another exemplary embodiment of the present invention. Referring now to FIG. 7, the probe device 101 according to the current exemplary embodiment is substantially identical to the probe device 100 of the previous embodiment, except that the probe device 101 further includes a transmitter beam former 122 in the pulse generator 120 and a receiver beam former 133 in the analog signal processor 130.

In the above exemplary embodiment, the server 300 performs the transmitter beam forming process and the receiver beam forming process, but in the current embodiment, the transmitter beam former 122 and the receiver beam former 133 included in the probe device 101 respectively perform the transmitter beam forming process and the receiver beam forming process. For example, the transmitter beam former 122 enables the pulser 121 to generate pulse signals to be applied to each of the conversion elements of the transducer 110 by considering the locations of the conversion elements and the focus point of the ultrasonic waves. The receiver beam former 133 performs a process of focusing to view reflection characteristics of a tissue at a desired location from a received ultrasonic signal.

In the current exemplary embodiment, since the transmitter and receiver beam forming processes are performed by the probe device 101, data according to the transmitter and receiver beam forming processes do not need to be transmitted and received through the first network 200, and thus an amount of data transmitted and received in the probe communicator 150 may be reduced.

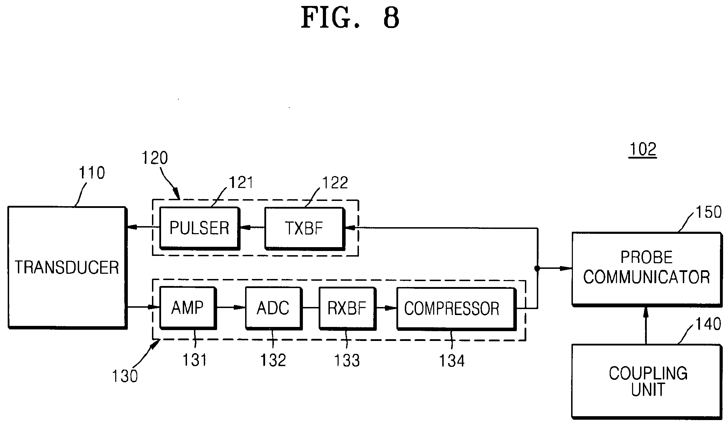

FIG. 8 is a block diagram of a probe device 102 according to another exemplary embodiment of the present invention. Referring to FIG. 8, the probe device 102 according to the current embodiment is substantially identical to the probe device 101 of FIG. 7, except that the probe device 102 further includes a compressor 134 in the analog signal processor 130. The compressor 134 compresses a data size of a digital echo signal generated by the analog signal processor 130.

In the previous exemplary embodiments, uncompressed echo data is transmitted to the server 300 from the probe devices 100 and 102, but the probe device 102 of the current exemplary embodiment transmits compressed data to the server 300. Thus, more pieces of information are substantially transmitted to the server 300 in real-time, and an ultrasound image having a high resolution may be obtained in real-time. A compression program can be used to compress the data.

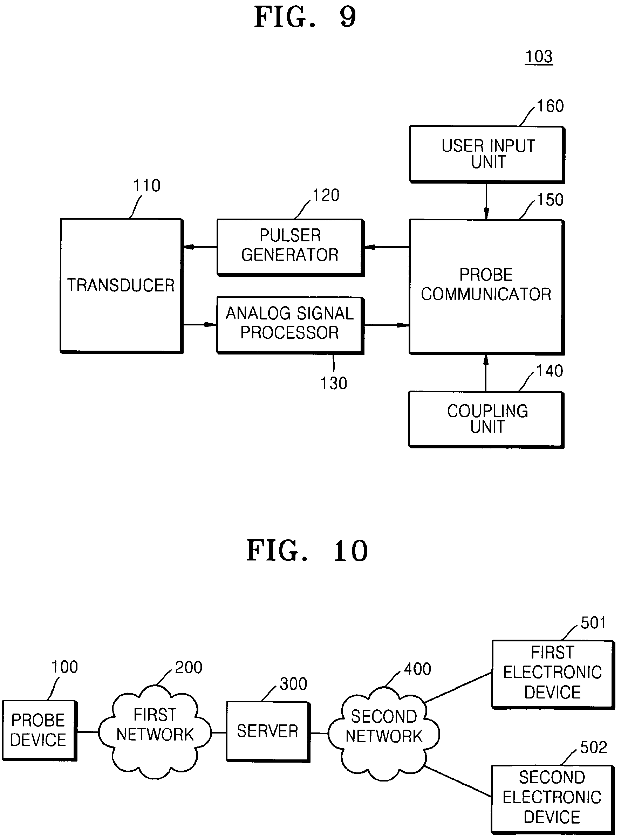

FIG. 9 is a block diagram of a probe device 103 according to another embodiment of the present invention. Referring now to FIG. 9, the probe device 103 is substantially identical to the probe devices 100, 101, and 102 of the previous embodiments, except that the probe device 103 further includes a user input unit 160. The user input unit 160 may be a key input unit for manipulating the probe device 103. In some cases, the user input unit 160 may provide a user interface of the ultrasound image diagnostic application executed in the server 300. As such, since the probe device 103 additionally includes the user input unit 160, the probe device 103 may be manipulated by using the probe device 103 itself. The user input may also comprise a portion of the touchscreen.

FIG. 10 is a diagram of a system for diagnosing an ultrasound image, according to still another exemplary embodiment of the present invention.

Referring now to FIG. 10, the system according to the current exemplary embodiment includes the probe device 100, the server 300, a first electronic device 501, and a second electronic device 502. In the current exemplary embodiment one system is formed as the first and second electronic devices 501 and 502 are combined with respect to one probe device 100. Of course, three or more electronic devices may be combined for one probe device 100. The first and second electronic devices 501 and 502 are substantially identical to the electronic device 500 described with reference to FIGS. 1 through 9, except that the first and second electronic devices 501 and 502 are combined to one probe device 100. In other words, mechanical structures of the first and second electronic devices 501 and 502 are substantially identical to that of the electronic device 500.

A cooperation relation between the first and second electronic devices 501 and 502 may vary.

For example, the ultrasound image diagnostic application executed in the server 300 may be controllable by any one of the first and second electronic devices 501 and 502. Alternatively, the ultrasound image diagnostic application may be controllable by both the first and second electronic devices 501 and 502. Since the ultrasound image diagnostic application not only generates the ultrasound image based on the echo signal from the probe device 100, but also controls the probe device 100, any one or both of the first and second electronic devices 501 and 502 may be used to control the probe device 100. Also, the ultrasound image or the user interface of the ultrasound image diagnostic application displayed on the first electronic device 501 may be manipulatable from the second electronic device 502 connected through the server 300, or alternatively, the ultrasound image or the user interface of the ultrasound image diagnostic application displayed on the second electronic device 502 may be manipulatable from the first electronic device 501 connected through the server 300.

For example, the first electronic device 501 may be adjacent to the probe device 100 and/or locally coupled to the first electronic device 501, and thus the first electronic device 501 may be connected to the server 300 and the ultrasound image diagnostic application of the server 300 may be executed before the operation of the probe device 100, i.e., before ultrasonic waves scan the person to be examined. For example, the probe device 100 and the first electronic device 501 may be operated by the same person. Meanwhile, the second electronic device 502 is remotely located from the probe device 100, i.e., the person to be examined, and the ultrasound image based on the echo signal obtained by the probe device 100 may be remotely viewed in real-time.

In more detail, the first electronic device 501 may be used by an emergency rescuer and the second electronic device 502 may be used by an image diagnostic expert at a hospital or the like. In this case, when the emergency rescuer executes the ultrasound image diagnostic application of the server 300 through the first electronic device 501 and scans a predetermined part of the person to be examined while interlocking the probe device 100 with the ultrasound image diagnostic application in an emergency, the remote image diagnostic expert may direct manipulation (for example, pointing out a part to be scanned) of the probe device 100 to the emergency rescuer through a communication unit such as a phone, while looking at an ultrasound image on a screen of the ultrasound image diagnostic application executed by the server 300 through the second electronic device 502 in real-time. If the ultrasound image or the user interface of the ultrasound image diagnostic application displayed on the first electronic device 501 is manipulatable from the second electronic device 502, the image diagnostic expert may directly manipulate the ultrasound image or other data displayed on the first electronic device 501 through the second electronic device 502. A remote configuration programs can be used as one possible example for device 502 to manipulate device 501. Also, the image diagnostic expert may select a mode of the ultrasound image (for example, a B mode, a D mode, a C mode, or an elastic mode) by manipulating a manipulation menu of the ultrasound image diagnostic application executed in the server 300 through the second electronic device 502. A third electronic device may be further included for a third person to view the ultrasound image through the third electronic device.

Next, operations of the system according to the current embodiment will be described.

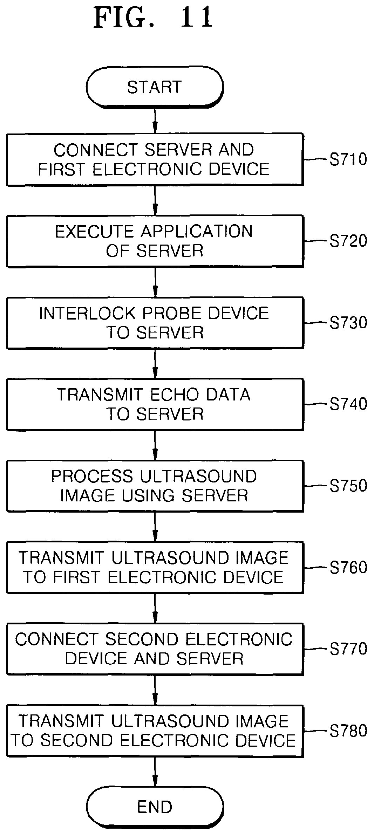

FIG. 11 is a flowchart illustrating a method of processing an ultrasound image, according to an exemplary embodiment of the present invention.

Referring now to FIGS. 10 and 11, the system of the current exemplary embodiment operates as follows. First, the first electronic device 501 connects to the server 300 in operation S710. The first electronic device 501 may be a terminal possessed by a medical image expert, such as a doctor, a nurse, a medical laboratory technologist, or an emergency rescuer, or a terminal possessed by a person to be examined. When a message requesting to execute the ultrasound image diagnostic application is transmitted from the first electronic device 501 to the server 300, the server 300 executes the ultrasound image diagnostic application in operation S720. User authentication may be performed when the first electronic device 501 is connected to the server 300 or when the ultrasound image diagnostic application is executed. Then, the probe device 100 is turned on, and is interlocked with the ultrasound image diagnostic application of the server 300, in operation S730. Then, the probe device 100 transmits digital echo data generated from reflected ultrasonic waves to the server 300 in operation S740. The ultrasound image diagnostic application of the server 300 generates ultrasound image data by using the echo data received from the probe device 100 in operation S750.

The server transmits the generated ultrasound image data to the first electronic device 501 in operation S760, and the ultrasound image is displayed on a display unit (refer to the display unit 520 of FIG. 4) of the first electronic device 501.

Meanwhile, the second electronic device 502 connects to the server 300 to execute the ultrasound image diagnostic application in operation S770. The second electronic device 502 may be remotely located from the probe device 100.

For example, the probe device 100 and the first electronic device 501 may be located outdoors or at home and used by an amateur, such as a patient or an emergency rescuer, and the second electronic device 502 may be located at a hospital and used by an expert, such as a doctor. When the second electronic device 502 executes the ultrasound image diagnostic application, the ultrasound image diagnostic application transmits the ultrasound image based on the echo data obtained from the probe device 100 to the second electronic device 502, and the second electronic device 502 displays the ultrasound image in operation S780. Furthermore, the server 300 may provide the ultrasound image diagnostic application to the second electronic device 502 so as to allow the second electronic device 502 to control the ultrasound image diagnostic application executed in the server 300. In addition, user authentication may be additionally performed when the second electronic device 502 is connected to the server 300 or the second electronic device 502 executes the ultrasound image diagnostic application.

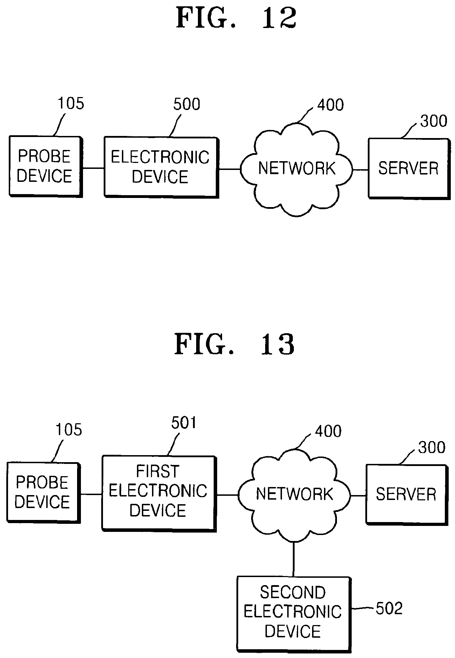

FIG. 12 is a diagram of a system for diagnosing an ultrasound image, according to another exemplary embodiment of the present invention.

Referring to FIG. 12, the system according to the current embodiment includes a probe device 105, a server 300, and an electronic device 500. In the current embodiment, the probe device 105 and the electronic device 500 are directly connected to each other and the probe device 105 connects to a network 400 through the electronic device 500. The probe device 105 includes a communication interface connectable to the electronic device 500, and may not include a communication module that directly connects to the network 400. As such, by using the communication interface that is low-priced, is light weighted, and has low power consumption instead of a high-priced communication module for the probe device 105, manufacturing costs of the probe device 105 may be reduced, a weight of the probe device 105 may be decreased, and power consumption of the probe device 105 may be decreased, thereby improving portability of the probe device 105.

The server 300 and the electronic device 500 of FIG. 12 may respectively be the server 300 and the electronic device 500 of FIGS. 3 and 4. The current embodiment is substantially identical to those described with reference to FIGS. 1 through 9, except that the probe device 105 connects to the network 400 through the electronic device 500.

For example, a probe communicator of the probe device 105 and a terminal communicator of the electronic device 500 may include a communication interface, such as a USB or a Bluetooth, and only the electronic device 500 may include a mobile communication module, a wired Internet module, a wireless Internet module, or a local area communication module, which connects to the network 400. In this case, the probe device 105 connects to the electronic device 500 wirelessly or via wires, and may connect to the server 300 through the electronic device 500.

FIG. 13 is a diagram of a system for diagnosing an ultrasound image, according to another exemplary embodiment of the present invention.

Referring to FIG. 13, the system according to the current embodiment includes a probe device 105, a server 300, a first electronic device 501, and a second electronic device 502. In the current embodiment, the probe device 105 and the first electronic device 501 are directly connected to each other, and the probe device 106 connects to the server 300 on a network 400 through the first electronic device 501. Also, the second electronic device 502 connects to the server 300 through the network 400. Of course, an electronic device may additionally connect to the server 300 through the network 400. The current embodiment is substantially identical to those described with reference to FIGS. 10 and 11, except that the probe device 105 connects to the network 400 through the first electronic device 501. Meanwhile, the first electronic device 501 may simply function only as a communication module of the probe device 105. In this case, the first electronic device 501 may be understood to be an external type of the probe communicator 150 of the probe device 100 described above with reference to FIG. 2.

According to the systems and the methods of the exemplary embodiments of the present invention, a high load image process is focused on the server such that the probe device or the electronic device does not require a high performance processing apparatus. Thus, the probe device or the electronic device can be compacted and slimmed down to improve portability and reduce manufacturing costs.

In the case where the first device and/or the second device may both constitute a mobile communication terminal, such as a user in a remote area using a probe 100 attached to or integrated with device 95 to provide the information to a medical expert via the second electronic device 500, the person probing and the medical expert can make or receive telephone communications to the person self-probing or being probed locally while a user of the second electronic device or another communication device may provide verbal feedback or instructions to the user of the device 95 instead of or in addition to the second electronic device manipulating the device 95. Such an exemplary embodiment is advantageous as the first user may not be a trained ultrasound technician and can receive instructions while directing the probe, for example, when the device has a speakerphone turned on. A person of skill in the art can appreciate that as some mobile communication terminals can access the Internet while making a voice call, which may or may not include voice over IP (VOIP), and the ultrasound can be activated and relaying results via a server or base station while speaking with a doctor, ultrasound technician, emergency medical member, which may be critically advantageous in the case of a serious injury. Also, according to the systems and the methods of the exemplary embodiments of the present invention, the time taken to start diagnosis by using high performance computing resources of the server is reduced, and thus a patient can be quickly diagnosed.

In addition, according to the systems and methods of the exemplary embodiments of the present invention, the user is able to view progress of a corresponding application by connecting to the system through the electronic device connected to the network, and thus spatial limitation is reduced and a limitation to a number of displays is reduced.

The above-described methods according to the present invention can be implemented in hardware, firmware or as software or computer code that is loaded into hardware such as a microprocessor and can be stored in a recording medium such as a CD ROM, an RAM, flash drive, a floppy disk, a hard disk, or a magneto-optical disk or computer code downloaded over a network originally stored on a remote recording medium or a non-transitory machine readable medium and to be stored on a local recording medium, so that the methods described herein can be rendered in such software that is stored on the recording medium using a general purpose computer and loaded into hardware such as a processor or microprocessor, or a special processor or in programmable or dedicated hardware, such as an ASIC or FPGA. As would be understood in the art, the computer, the processor, microprocessor or controller are hardware elements and the claims are to be interpreted with such elements comprising hardware, and not to be interpreted under the broadest reasonable interpretation as being pure software that is outside the scope of a statutory invention. The programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein.

The invention can also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, etc. The computer readable recording medium can also be distributed over network coupled computer systems so that the computer readable code is stored and executed in a distributed fashion. Moreover, all of the modules described herein constitute hardware modules that such as microprocessors that can be configured with software or firmware, as the claimed invention in accordance with the appended claims are directed to statutory subject matter.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A system for performing an ultrasound imaging operation, the system comprising:
- a server configured as an ultrasound diagnostic system,
- a probe device comprising:
- a pulse generator (120) comprising a pulser (121) configured to generate a pulse signal;
- a transducer (110) configured to convert the generated pulse signal into ultrasonic waves for transmission toward a body, and to convert ultrasonic waves that are reflected back from the body into an electrical signal;
- a signal processor (130) for generating an echo signal of the electrical signal converted from the reflected ultrasonic waves by the transducer; and
- a probe communicator (150);
wherein the probe communicator is configured for communicating through a first network (200) with the server (300),
wherein the probe communicator (100, 103, 105) is configured to transmit the echo signal to the server, through the first network (200), wherein the server comprises:
- a server communicator (310) configured to communicate with the probe device through the first network (200) and an electronic device (500) through the first network (200) or a second network (400);
- a data storage unit (330) configured to store an ultrasound image diagnostic application; and
- an operation unit (320) configured to execute the ultrasound image diagnostic application in response to a request by the electronic device,
wherein the operation unit is configured to generate ultrasound image data utilizing the echo signal transmitted from the probe device, and to transmit the generated ultrasound image data to the electronic device,
wherein the probe communicator comprises at least one of a mobile communication module directly connecting to the first network, a wireless Internet module, a wired Internet module, and a local area communication module,
wherein the transducer comprises a plurality of conversion elements configured to convert and transmit a pulse signal generated by the pulse generator to ultrasonic waves and to convert reflected ultrasonic waves to an electronic signal,
wherein the ultrasound diagnostic application executed by the server is configured
- to execute a transmitter beam forming process to focus ultrasonic waves output from the transducer to view reflection characteristics of a tissue at a desired location and to determine pulse signals to be applied to each of the conversion elements of the transducer by considering locations of the conversion elements and a focus point of the output ultrasonic waves; and/or
- to execute a receiver beam forming process to focus a received ultrasound echo signal to view reflection characteristics of a tissue at a desired location.

2. The system of claim 1, wherein the probe communicator is configured to transmit an identification information of the probe device to the server when the probe device connects to the server.

3. The system of claim 2, wherein the server communicator is configured to receive the identification information of the probe device from the probe device, and the ultrasound image diagnostic application is configured to display the identification information of communicable probe devices on the display unit of the electronic device enabling a user to select a particular probe from the plurality of communicable ultrasound probe devices using the ultrasound probe device identification information.

4. The system of claim 1, 2 or 3, further comprising a user input unit is configured to receive input commands.

5. The system of any one of the preceding claims, further comprising an electronic device comprising a display unit configured to receive through the first network or second network and to display the ultrasound image data generated by the ultrasound image diagnostic application of the server.

6. The system of claim 5, wherein the server transmits a manipulation menu for selecting a mode among a plurality of modes for processing the ultrasound image to the electronic device, and further transmits the generated ultrasound image data and
the display unit of the electronic device displays the plurality of modes for processing the ultrasound image so that the user selects a mode among the plurality of modes, and further displays the ultrasound image.

7. The system of claim 5, wherein the probe device and the electronic device are configured in such a way that operation of the probe device is controllable by the electronic device.

8. The system of claim 7, wherein the server transmits manipulation menu information about controlling the probe device to the electronic device so that the user controls the probe device by manipulating the electronic device, and further transmits the generated ultrasound image data, and
the display unit of the electronic device displays the manipulation menu information about controlling the probe device so that the user controls the probe device by manipulating the electronic device, and further displays the ultrasound image.

9. The system of any one of the claims 5-8, wherein the electronic device comprises a first electronic device and a second electronic device, which are configured to respectively receive and display ultrasound image data generated by the ultrasound image diagnostic application of the server.

10. The system of claim 9, wherein the ultrasound image diagnostic application executed by the server is controlled by at least one of the first and second electronic devices.

11. The system of claim 9, wherein the first and second electronic device are configured in such a way that the ultrasound image data or the user interface of the ultrasound image diagnostic application displayed on a display of the first electronic device is manipulatable by the second electronic device connected through the server.

12. The system of any one of claims 5-11, wherein the electronic device comprises a mobile terminal connected to a wireless network or a desktop connected to a wired or wireless network and a display configured to display the ultrasound image data generated by the ultrasound image diagnostic application of the server.

13. The system of any of the preceding claims, wherein the electronic device comprises a mobile terminal connected to a wireless network or a desktop connected to a wired or wireless network, wherein the mobile terminal preferably comprises a mobile phone, a smart phone, a touch pad, a laptop, a digital broadcasting terminal, a personal digital assistant (PDA), a portable multimedia player (PMP), a navigation device, a tablet personal computer (PC) or a remote controller.

14. A method of performing an ultrasound imaging operation through the system of any one of claims 1-13, the method comprising:
receiving (S610, S710), by the server, a request for executing the ultrasound image diagnostic application transmitted from the electronic device through the first network (200) or second network (400);
executing (S620, S720), by the server, the ultrasound image diagnostic application in response to the request from the electronic device;
receiving (S650, S740), by the server, an echo signal of a material to be examined generated by the probe device and transmitted by the probe device to the server;
generating (S670, S750), by the server, ultrasound image data using the echo signal received; and
transmitting (S670, S760, S780), by the server, the generated ultrasound image data to the electronic device through the network.

15. The method of claim 14, wherein the echo signal received by the server is compressed in data size.

16. The method of claim 14 or 15, further comprising transmitting, by the server, a user interface of the ultrasound image diagnostic application executed by the server to the electronic device.

## Patentansprüche

1. System zur Durchführung einer Ultraschallbildfunktion, wobei das System umfasst:
- einen Server, der als ein Ultraschalldiagnosesystem konfiguriert ist;
- eine Sondenvorrichtung, die umfasst:
- einen Impulsgenerator (120), der einen Pulsgeber (121) umfasst, der dazu konfiguriert ist, ein Impulssignal zu erzeugen;
- einen Wandler (110), der dazu konfiguriert ist, das erzeugte Impulssignal in Ultraschallwellen, die auf einen Körper übertragen werden, umzuwandeln, und um Ultraschallwellen, die von dem Körper zurück reflektiert werden, in ein elektrisches Signal umzuwandeln;
- ein Signalverarbeitungsgerät (130) zur Erzeugung eines Echosignals von dem elektrischen Signal, das von dem Wandler aus dem reflektierten Ultraschallsignal umgewandelt wurde; und
- einen Sondenkommunikator (150);
wobei der Sondenkommunikator dazu konfiguriert ist, durch ein erstes Netzwerk (200) mit dem Server (300) zu kommunizieren,
wobei der Sondenkommunikator (100, 103, 105) dazu konfiguriert ist, das Echosignal durch das erste Netzwerk (200) an den Server zu übertragen, wobei der Server umfasst:
- einen Serverkommunikator (310), der dazu konfiguriert ist, mit der Sondenvorrichtung über das erste Netzwerk (200) zu kommunizieren und mit einem elektronischen Gerät (500) über das erste Netzwerk (200) oder ein zweites Netzwerk (400) zu kommunizieren;
- eine Datenspeicherungseinheit (330), die dazu konfiguriert ist, eine Ultraschallbilddiagnoseanwendung zu speichern; und
- eine Betriebseinheit (320), die dazu konfiguriert ist, die Ultraschallbilddiagnoseanwendung als Reaktion auf eine Anforderung durch das elektronische Gerät auszuführen,
wobei die Betriebseinheit dazu konfiguriert ist, Ultraschallbilddaten mit Hilfe des von der Sondenvorrichtung übertragenen Echosignals zu erzeugen und die erzeugten Ultraschallbilddaten an das elektronische Gerät zu übertragen.
wobei der Sondenkommunikator wenigstens ein Modul aus den folgenden Modulen umfasst: ein mobiles Kommunikationsmodul, das direkt mit dem ersten Netzwerk verbunden ist, ein drahtloses Internetmodul, ein kabelgebundenes Internetmodul und ein lokales Kommunikationsmodul,
wobei der Wandler eine Mehrzahl von Umwandlungselementen umfasst, die dazu konfiguriert sind, ein von dem Impulsgenerator erzeugtes Impulssignal in Ultraschallwellen umzuwandeln und zu senden, und um reflektierte Ultraschallwellen in ein elektrisches Signal umzuwandeln,
wobei die von dem Server ausgeführte Ultraschallbilddiagnoseanwendung dazu konfiguriert ist,
- einen Übertragungsstrahlbildungsprozess auszuführen, um Ultraschallwellen, die von dem Wandler ausgegeben wurden, zu fokussierten, um Reflexionscharakteristika eines Gewebes an einer gewünschten Stelle zu betrachten, und um Impulssignale zu bestimmen, die an jedes der Umwandlungselemente des Wandlers angewendet werden sollen, durch in Betracht ziehen der Umwandlungselemente und des Fokuspunktes der ausgegebenen Ultraschallwellen; und/oder
- einen Empfängerstrahlbildungsprozess auszuführen, um ein empfangenes Echosignal zu fokussierten, um Reflexionscharakteristika eines Gewebes an einer gewünschten Stelle zu betrachten.

2. Sondenvorrichtung gemäß Anspruch 1, wobei der Sondenkommunikator dazu konfiguriert ist, eine Identifikationsinformation der Sondenvorrichtung an den Server zu übertragen, wenn die Sonde mit dem Server verbunden wird.

3. System gemäß Anspruch 2, wobei der Serverkommunikator dazu konfiguriert ist die Identifikationsinformation der Sondenvorrichtung von der Sondenvorrichtung zu empfangen, und die Ultraschallbilddiagnoseanwendung dazu konfiguriert ist, die Identifikationsinformation kommunikationsfähiger Sondenvorrichtungen auf der Anzeigeeinheit des elektronischen Geräts anzuzeigen, wodurch es einem Benutzer ermöglicht wird, eine bestimmte Sonde aus der Mehrzahl von kommunikationsfähigen Ultraschallsondenvorrichtungen mit Hilfe der Identifikationsinformation der Sondenvorrichtung auszuwählen.

4. System gemäß Anspruch 1, 2 oder 3, das weiterhin eine Benutzereingabeeinheit umfasst, die dazu konfiguriert ist, Eingabebefehle zu empfangen.

5. System gemäß einem der vorangegangenen Ansprüche, das weiterhin ein elektronisches Gerät umfasst, das eine Anzeigeeinheit umfasst, die dazu konfiguriert ist, die von der Ultraschallbilddiagnoseanwendung erzeugten Ultraschallbilddaten durch das erste oder zweite Netzwerk zu empfangen und anzuzeigen.

6. System gemäß Anspruch 5, wobei der Server ein Manipulationsmenü zum Auswählen eines Modus unter einer Mehrzahl von Modi zum Verarbeiten des Ultraschallbilds an das elektronische Gerät überträgt, und des Weiteren die erzeugten Ultraschallbilddaten überträgt, und
wobei die Anzeigeeinheit des elektronischen Geräts die Mehrzahl von Modi zur Verarbeitung des Ultraschallbilds anzeigt, so dass der Benutzer einen Modus unter der Mehrzahl von Modi auswählen kann, und des Weiteren das Ultraschallbild anzeigt.

7. System gemäß Anspruch 5, wobei die Sondenvorrichtung und das elektronische Gerät auf solch eine Weise konfiguriert sind, dass ein Betrieb der Sondervorrichtung durch das elektronische Gerät gesteuert werden kann.

8. System gemäß Anspruch 7, wobei der Server Informationen zu dem Manipulationsmenü zur Steuerung der Sondenvorrichtung an das elektronische Gerät sendet, damit der Benutzer die Sondenvorrichtung durch Manipulieren des elektronische Geräts steuern kann, und des Weiteren die erzeugten Ultraschallbilddaten überträgt, und
wobei die Anzeigeeinheit des elektronischen Geräts die Informationen zu dem Manipulationsmenü zur Steuerung der Sondenvorrichtung anzeigt, damit der Benutzer die Sondenvorrichtung durch Manipulieren des elektronische Geräts steuern kann, und des Weiteren die erzeugten Ultraschallbilddaten anzeigt.

9. System gemäß einem der Ansprüche 5 bis 8, wobei das elektronische Gerät ein erstes elektronisches Gerät und ein zweites elektronisches Gerät umfasst, die dazu konfiguriert sind, jeweils von der Ultraschallbilddiagnoseanwendung auf dem Server erzeugte Ultraschallbilddaten zu empfangen und anzuzeigen.

10. System gemäß Anspruch 9, wobei die von dem Server ausgeführte Ultraschallbilddiagnoseanwendung von dem ersten und/oder dem zweiten elektronischen Gerät gesteuert wird.

11. System gemäß Anspruch 9, wobei das erste elektronische Gerät und das zweite elektronische Gerät auf solch eine Weise konfiguriert sind, dass die Ultraschallbilddaten oder die Benutzeroberfläche der Ultraschallbilddiagnoseanwendung auf der Anzeige des ersten elektronischen Gerätes von dem zweiten elektronischen Gerät, das durch den Server verbunden ist, bedient werden können.

12. System gemäß einem der Ansprüche 5 bis 11, wobei das elektronische Gerät einen mobilen Terminal umfasst, der mit einem drahtlosen Netzwerk verbunden ist, oder einen Desktop, der mit einem kabelgebundenen oder drahtlosen Netzwerk verbunden ist, und eine Anzeige, die dazu konfiguriert ist, die von der Ultraschallbilddiagnoseanwendung auf dem Server erzeugten Ultraschallbilddaten anzuzeigen.

13. System gemäß einem der vorangegangenen Ansprüche, wobei das elektronische Gerät einen mobilen Terminal umfasst, der mit einem drahtlosen Netzwerk verbunden ist, oder einen Desktop, der mit einem kabelgebundenen oder drahtlosen Netzwerk verbunden ist, wobei der mobile Terminal vorzugsweise ein Mobiltelefon, ein Smartphone, ein Touchpad, einen Laptop, einen digitalen Rundfunk-Terminal, einen Persönlichen Digitalen Assistenten (PDA), einen tragbaren Mediaplayer (PMP, Portable Media Player), ein Navigationsgerät, einen Tablet-PC oder eine Fernbedienung umfasst.

14. Verfahren zur Durchführung einer Ultraschallbildfunktion durch das System gemäß einem der Ansprüche 1 bis 13, wobei das Verfahren umfasst:
Empfangen (S610, S710), durch den Server, einer Anforderung zur Durchführung der Ultraschallbilddiagnoseanwendung, die von dem elektronischen Gerät über das erste Netzwerk (200) oder über das zweite Netzwerk (400) übertragen wurde;
Ausführen (S620, S720), durch den Server, der Ultraschallbilddiagnoseanwendung als Reaktion auf die Anforderung durch das elektronische Gerät;
Empfangen (S650, S740), durch den Server, eines Echosignals von einem zu untersuchenden Material, das von der Sondenvorrichtung erzeugt wurde und von der Sondenvorrichtung an den Server übertragen wurde;
Erzeugen (S670, S750), durch den Server, von Ultraschallbilddaten mit Hilfe des empfangenen Echosignals; und
Übertragen (S670, S760, S780), durch den Server, der erzeugten Ultraschallbilddaten über das Netzwerk an das elektronische Gerät.

15. Verfahren gemäß Anspruch 14, wobei das von dem Server empfangene Echosignal in Datengröße komprimiert wird.

16. Verfahren gemäß Anspruch 14 oder 15, das weiterhin das Übertragen, durch den Server, einer Benutzeroberfläche der auf dem Server ausgeführten Ultraschallbilddiagnoseanwendung an das elektronische Gerät umfasst.

## Revendications

1. Système visant à réaliser une opération d'imagerie ultrasonore, le système comprenant :
- un serveur, configuré sous la forme d'un système de diagnostic ultrasonore ;
- un dispositif de sonde comprenant :
- un générateur d'impulsions (120) comprenant un pulsateur (121) configuré pour générer un signal d'impulsion ;
- un transducteur (110) configuré pour convertir le signal d'impulsion généré en ondes ultrasonores destinées à être transmises vers un corps, et pour convertir les ondes ultrasonores qui sont réfléchies par le corps en un signal électrique ;
- un processeur de signaux (130) destiné à générer un signal d'écho du signal électrique converti par le transducteur à partir des ondes ultrasonores réfléchies ; et
- un communicateur de sonde (150) ;
dans lequel le communicateur de sonde est configuré pour communiquer, par le biais d'un premier réseau (200), avec le serveur (300),
dans lequel le communicateur de sonde (100, 103, 105) est configuré pour transmettre le signal d'écho auprès du serveur, par le biais du premier réseau (200), le serveur comprenant :
- un communicateur de serveur (310) configuré pour communiquer avec le dispositif de sonde par le biais du premier réseau (200) et avec un appareil électronique (500) par le biais du premier réseau (200) ou d'un second réseau (400) ;
- une unité de stockage de données (330) configurée pour stocker une application de diagnostic d'image ultrasonore ; et
- une unité fonctionnelle (320) configurée pour exécuter l'application de diagnostic d'image ultrasonore en réponse à une demande provenant de l'appareil électronique,
dans lequel l'unité fonctionnelle est configurée pour générer des données d'image ultrasonore au moyen du signal d'écho transmis par le dispositif de sonde, et pour transmettre les données d'image ultrasonore générées auprès de l'appareil électronique,
dans lequel le communicateur de sonde comprend au moins un module parmi : un module de communication mobile en connexion directe avec le premier réseau, un module Internet sans fil, un module Internet filaire et un module de communication en réseau local,
dans lequel le transducteur comprend une pluralité d'éléments de conversion configurés pour convertir et transmettre un signal d'impulsion généré par le générateur d'impulsions en ondes ultrasonores et pour convertir les ondes ultrasonores réfléchies en un signal électronique,
dans lequel l'application de diagnostic d'image ultrasonore exécutée par le serveur est configurée pour :
- exécuter un processus de formation de faisceau de transmission pour concentrer les ondes ultrasonores transmises par le transducteur afin de visualiser les caractéristiques de réflexion d'un tissu à un emplacement souhaité et pour déterminer des signaux d'impulsion devant être appliqués à chacun des éléments de conversion du transducteur en tenant compte des emplacements des éléments de conversion et d'un point de concentration des ondes ultrasonores transmises ; et/ou
- exécuter un processus de formation de faisceau de réception pour concentrer un signal d'écho ultrasonore reçu afin de visualiser les caractéristiques de réflexion d'un tissu à un emplacement souhaité.

2. Système selon la revendication 1, dans lequel le communicateur de sonde est configuré pour transmettre des informations d'identification relatives au dispositif de sonde auprès du serveur lorsque la sonde se connecte au serveur.

3. Système selon la revendication 2, dans lequel le communicateur de serveur est configuré pour recevoir les informations d'identification relatives au dispositif de sonde provenant du dispositif de sonde, et l'application de diagnostic d'image ultrasonore est configurée pour afficher, sur l'unité d'affichage de l'appareil électronique, les informations d'identification des dispositifs de sonde aptes à communiquer, permettant à un utilisateur de choisir une sonde particulière parmi la pluralité de dispositifs de sonde ultrasonores aptes à communiquer, au moyen des informations d'identification des dispositifs de sonde ultrasonores.

4. Système selon la revendication 1, 2 ou 3, comprenant en outre une unité d'entrée utilisateur qui est configurée pour recevoir des commandes d'entrée.

5. Système selon l'une quelconque des revendications précédentes, comprenant en outre un appareil électronique comprenant une unité d' affichage configuré pour recevoir, par le biais du premier réseau ou du second réseau, et pour afficher, les données d'image ultrasonore générées par l'application de diagnostic d'image ultrasonore du serveur.

6. Système selon la revendication 5, dans lequel le serveur transmet un menu de manipulation permettant de choisir un mode parmi une pluralité de modes visant au traitement de l'image ultrasonore par l'appareil électronique, et transmet en outre les données d'image ultrasonore générées, et
l'unité d'affichage de l'appareil électronique affiche la pluralité de modes visant au traitement de l'image ultrasonore de manière que l'utilisateur puisse choisir un mode parmi la pluralité de modes, et affiche en outre l'image ultrasonore.

7. Système selon la revendication 5, dans lequel le dispositif de sonde et l'appareil électronique sont configurés de telle manière que le fonctionnement du dispositif de sonde peut être contrôlé par l'appareil électronique.

8. Système selon la revendication 7, dans lequel le serveur transmet des informations relatives au menu de manipulation concernant le contrôle du dispositif de sonde auprès de l'appareil électronique afin que l'utilisateur puisse contrôler le dispositif de sonde en manipulant l'appareil électronique, et transmet en outre les données d'image ultrasonore générées, et
l'unité d'affichage de l'appareil électronique affiche les informations relatives au menu de manipulation concernant le contrôle du dispositif de sonde afin que l'utilisateur puisse contrôler le dispositif de sonde en manipulant l'appareil électronique, et affiche en outre l'image ultrasonore.

9. Système selon l'une quelconque des revendications 5 à 8, dans lequel l'appareil électronique comprend un premier appareil électronique et un second appareil électronique, lesquels sont respectivement configurés pour recevoir et pour afficher les données d'image ultrasonore générées par l'application de diagnostic d'image ultrasonore du serveur.

10. Système selon la revendication 9, dans lequel l'application de diagnostic d'image ultrasonore exécutée par le serveur est contrôlée par au moins un appareil parmi le premier et le second appareil électronique.

11. Système selon la revendication 9, dans lequel le premier et le second appareil électronique sont configurés de telle manière que les données d'image ultrasonore ou l'interface utilisateur de l'application de diagnostic d'image ultrasonore apparaissant sur un dispositif d'affichage du premier appareil électronique puissent être manipulées par le second appareil électronique connecté par le biais du serveur.

12. Système selon l'une quelconque des revendications 5 à 11, dans lequel l'appareil électronique comprend un terminal mobile connecté à un réseau sans fil ou à un ordinateur de bureau connecté à un réseau filaire ou sans fil et un dispositif d'affichage configuré pour afficher les données d'image ultrasonore générées par l'application de diagnostic d'image ultrasonore du serveur.

13. Système selon l'une quelconque des revendications précédentes, dans lequel l'appareil électronique comprend un terminal mobile connecté à un réseau sans fil ou un ordinateur de bureau connecté à un réseau filaire ou sans fil, ledit terminal mobile comprenant de préférence un téléphone mobile, un smartphone, un écran tactile, un ordinateur portable, un terminal de diffusion numérique, un assistant numérique personnel, un lecteur multimédia portable, un appareil de navigation, un ordinateur personnel (PC) sous forme de tablette ou une télécommande.

14. Procédé de réalisation d'une opération d'imagerie ultrasonore par le biais du système selon l'une quelconque des revendications 1 à 13, le procédé comprenant :
la réception (S610, S710), par le serveur, d'une demande d'exécution de l'application de diagnostic d'image ultrasonore transmise par l'appareil électronique par le biais du premier réseau (200) ou du second réseau (400) ;
l'exécution (S620, S720), par le serveur, de l'application de diagnostic d'image ultrasonore en réponse à la demande provenant de l'appareil électronique ;
la réception (S650, S740), par le serveur, d'un signal d'écho d'un matériau destiné à être examiné généré par le dispositif de sonde et transmis par le dispositif de sonde auprès du serveur ;
la génération (S670, S750), par le serveur, de données d'image ultrasonore au moyen du signal d'écho reçu ; et
la transmission (S670, S760, S780), par le serveur, des données d'image ultrasonore générées auprès de l'appareil électronique, par le biais du réseau.

15. Procédé selon la revendication 14, dans lequel le signal d'écho reçu par le serveur est comprimé en termes de taille des données.

16. Procédé selon la revendication 14 ou 15, comprenant en outre la transmission, par le serveur, d'une interface utilisateur de l'application de diagnostic d'image ultrasonore exécutée par le serveur auprès de l'appareil électronique.
